# EUROPEAN PATENT SPECIFICATION

(11) **EP 1 726 953 B1**
(45) Date of publication and mention of the grant of the patent: **20.08.2008**
(21) Application number: 06113906.9
(22) Date of filing: 15.05.2006
(51) Int. Cl.: G01N 33/50

(54) **Biomarker for response monitoring of CDK inhibitors in the clinic**
Biomarker zur Reaktionsüberwachung von CdK-Hemmern in der Klinik
Biomarqueur pour surveiller la réponse des inhibiteurs CDK dans la clinique

(30) Priority: 25.05.2005 US 684502 P
(43) Date of publication of application: 29.11.2006
(73) Proprietor: F. Hoffmann-La Roche AG, 4070 Basel (CH)
(72) Inventor: DePinto, Wanda Eve, Basking Ridge, NJ 07006 (US); Yin, Xuefeng, Westfield, NJ 07090 (US)

(56) References cited:
- EP-A- 1 207 395
- US-A1- 2003 166 016
- FRY D W ET AL: "Specific inhibition of cyclin-dependent kinase 4/6 by PD 0332991 and associated antitumor activity in human tumor xenografts" MOLECULAR CANCER THERAPEUTICS, AMERICAN ASSOCIATION OF CANCER RESEARCH, US, vol. 3, no. 11, November 2004 (2004-11), pages 1427-1438, XP002333887 ISSN: 1535-7163
- DATABASE BIOSIS [Online] BIOSCIENCES INFORMATION SERVICE, PHILADELPHIA, PA, US; 2004, MCCUE JESICA ET AL: "Cigarette Smoke Compounds Block Lymphocytes in the G0 Phase of the Cell Cycle" XP002399258 Database accession no. PREV200400287175
- WHITTAKER S R ET AL: "The Cyclin-dependent kinase inhibitor CYC202 (R-roscovitine) inhibits retinoblastoma protein phosphorylation, causes loss of Cyclin D1, and activates the mitogen-activated protein kinase pathway" CANCER RESEARCH, AMERICAN ASSOCIATION FOR CANCER RESEARCH, BALTIMORE, MD, US, vol. 64, no. 1, 1 January 2004 (2004-01-01), pages 262-272, XP002290632 ISSN: 0008-5472
- EZHEVSKY ET AL: "Differential regulation of retinoblastoma tumor suppressor protein by G1 cyclin-dependent kinase complex in vivo" MOLECULAR AND CELLULAR BIOLOGY, vol. 21, no. 14, July 2001 (2001-07), pages 4773-4784, XP002399145 ISSN: 0270-7306
- WADLER SCOTT: "Perspectives for cancer therapies with cdk2 inhibitors" December 2001 (2001-12), DRUG RESISTANCE UPDATES, VOL. 4, NR. 6, PAGE(S) 347-367 , XP002399147 ISSN: 1368-7646 * pages 350-351 *
- CLASSON M ET AL: "THE RETINOBLASTOMA TUMOUR SUPPRESSOR IN DEVELOPMENT AND CANCER" December 2002 (2002-12), NATURE REVIEWS. CANCER, NATUR PUBLISHING GROUP, LONDON, GB, PAGE(S) 910-917 , XP008044074 ISSN: 1474-175X * figure 1 *

## Description

The present invention relates generally to the field of pharmacodynamics and, more specifically, to a pharmacodynamic biomarker (inhibition of Rb phosphorylation) whose expression pattern in surrogate tissue correlates with a response of cells and tumors to treatment with one or more cdk inhibiting agents.

Uncontrolled proliferation is a hallmark of cancer cells. This proliferation seems to originate from the accumulation of defects in the cell cycle progression. These defects can result in the loss of checkpoint control and/or the inappropriate activation of the drivers of cell cycle progression, the cyclin-dependent kinases (referred to herein as "cdks" or "CDKs"). Misregulation of cdk function occurs with high frequency in major solid tumor types (including breast, colon, ovarian, prostate, and NSCL carcinomas). Therefore, inhibitors of cdks and cell cycle progression have the potential to fill a large therapeutic needThe cdks are serine/threonine protein kinases that are the driving force behind the cell cycle and cell proliferation. Cdks are multisubunit enzymes composed of at least a catalytic subunit and a regulatory (cyclin) subunit (Organ, D. O., Nature 1995; 374:131-134). To date, nine cdks (cdk1 through cdk9) and eleven cyclin subunits have been identified which can form in excess of thirteen active kinase complexes (J Gould, K. L., (1994) in Protein Kinases (Woodgett, J. R., ed), pp. 149-1766, Oxford University Press, Oxford). In normal cells, many of these enzymes can be categorized as G1, S, or G2/M phase enzymes which perform distinct roles in cell cycle progression (van den Heuvel, S., and Harlow, E., Science 1993; 262: 2050-2054). Cdks phosphorylate and modulate the activity of a variety of cellular proteins that include tumor suppressors (e.g., RB, p53), transcription factors (e.g., E2F-DP1, RNA pol II), replication factors (e.g., DNA pol α, replication protein A), and organizational factors which influence cellular and chromatin structures (e.g., Histone HI, lamin A, MPA4) (Nigg, E. A., Trends in Cell Biology 1993; 3:296-201; Rickert, P., et al, Oncogene 1996; 12:2631-2640; Dynlacht, B. D. et al, Mol Cell Biol. 1997; 17:3867-3875; Ookata, K. et al., Biochemistry 1997; 36:15873-15883).

Cdk activity is regulated through a variety of co-ordinated mechanisms, which include cell cycle dependent transcription and translation, cell cycle dependent proteolysis, subcellular localization, post-translational modifications, and interaction HR/24.03.2006 with cdk inhibitor proteins (referred to herein as "CDKi s") (Pines, J., and Hunter, T., Cell 1989; 58:833-846; King, R. W. et al, Science 1996; 274:1652-1659; Li, J. et al., Proc Natl Acad Sci USA 1997; 94:502-507; Fraetta, G., and Beach, D., Cell 1988; 54:17-26; Harper, J.W., Cancer Surv 1997; 29:91-107). It is through these mechanisms that cell cycle checkpoints are constructed. This realization that checkpoint control is implemented through the regulation of cdk function has made the cdks and their regulatory pathways compelling targets for the development of chemotherapeutic agents.In the early clinical development of anti-cancer agents, clinical trials are typically designed to evaluate the safety, tolerability, and pharmacokinetics, as well as to identify a suitable dose and schedule for further clinical evaluation. Increasingly, there is a need to also evaluate the pharmacologic effects of novel agents in early clinical trials, particularly in cases where dosing to maximum tolerated disease may not be appropriate. As a result, there is considerable interest in identifying a pharmacodynamic (PD) biomarker that correlates with the pharmacologic modulation of a tumor target. These PD biomarkers may be tumor-specific, but ideally should also be expressed in accessible surrogate tissues such as skin or peripheral blood cells, such as human peripheral blood mononuclear cells (PBMCs).Rb is a physiologically relevant substrate for the cyclin dependent kinases (CDKs). Rb (retinoblastoma protein) is a tumor suppressor. Activity of the Rb is regulated by phosphorylation at serine and threonine residues by the CDKs. As is known in the art, CDK inhibitors inhibit phosphorylation of the Rb substrate (such as in cell lines and tumor xenografts treated with CDKi s) and consequently this leads to cell cycle and growth arrest. Inhibition of Rb phosphorylation in cells has been reported for CYC 202 (r-Roscovitine), flavopirdol, and the CDK inhibitor from Bristol Myers Squibb, BMS-387032 (Whittaker, S.R. et al., Cancer Research 2004; 64:262-272, Yue, E.W. et al, Journal of Medicinal Chemistry 2002; 45:5233-5248). Inhibition of Rb phosphorylation in cells and in tumor xenografts has also been shown with the CDK inhibitor from Pfizer, PD 0332991 (Fry, D. W. et al, Molecular Cancer Therapeutics 2004: 3:1427-1437).

However, tumor tissue is not easily accessible for sampling, creating the need for a surrogate tissue that could be used as a substitute or replacement for the tumor tissue. Inhibition of Rb phosphorylation in a surrogate tissue, and specifically in peripheral blood mononuclear cells (PBMC's), has not been demonstrated in the art in either the preclinical setting or in the clinic with any of the CDK inhibitors currently in development. In a phase 1 trial of flavopiridol tested in combination with docetaxel in patients with metastatic tumors, phosphorylated Rb in 10 paired tumor and buccal mucosa biopsies was examined by immunohistochemistry (Tan, A.R. et al., Clinical Cancer Research 2004; 10:5038-5047). Although pRb somewhat decreased in buccal mucosa biopsies post treatment, no significant changes in pRb were detected in 6 paired tumors (viz-a-viz the biopsies). Due to the small sample size and unavailability of tumor tissue from the 3 partial responders, no definitive conclusion could be made regarding use of buccal mucosa tissue as a surrogate for predicting antitumor activity of flavopiridol.

Fry DW et al., (Mol Cancer Thera, 2004; 3(11) p. 1427-1438) describe the antitumour effect of the Cdk4 inhibitor PD 0332991 in human tumour xenograft model. PD 0332991 leads to a significant reduction of phospho Ser780 on Rb in tumour tissue (p. 1434, right column) of animals to which PD0332991 was administered. An antitumour activity of PD 0332991 was only detected at doses where there was sustained and significant reduction of phosphorylation of Ser780 on Rb in tumour tissue samples.

US 2003/166016 discloses a method for measuring the activity of cyclin dependent kinases (CDKs) in a sample based upon the quantitation of CDK phosphorylated Rb protein. The method uses a anti-Rb antibody which specifically recognises a CDK phosphorylated Rb protein.

Mccue J et al, (FASEB meeting on experimental biology, conference abstract, April 17-21, 2004) show that Cdk6 and Cdk4 activity (measured by in vitro phosphorylation of pRb substrate) is reduced in primary PBMCells exposed to hydroquinone and catechol (two major phenolic components of cigarette tar).

Thus, there remains a need to identify biomarkers whose expression patterns correlate with a response of cells to treatment with one or more cdk modulating agents, in surrogate tissues.

The instant invention provides an ex-vivo method for testing whether a mammal will respond therapeutically to a method of treating cancer comprising administering an agent that inhibits cdk activity, wherein the testing method comprises: a) activating a first amount of peripheral blood mononuclear cells of a mammal; b) measuring the level of the phosphorylation of retinoblastoma protein (Rb) at putative phosphorylation sites in the first amount of peripheral blood mononuclear cells of the mammal; c) activating a second amount of peripheral blood mononuclear cells of a mammal and simultaneously exposing the second amount of peripheral blood mononuclear cells of the mammal to the agent that inhibits cdk activity; d) measuring the level of the phosphorylation of retinoblastoma protein (Rb) at the putative phosphorylation sites in the second amount of peripheral blood mononuclear cells of the mammal, wherein a difference in the level of the phosphorylation of retinoblastoma protein measured in step d) compared to the level of the phosphorylation of retinoblastoma protein measured in step b) indicates that the mammal may respond therapeutically to the method of treating cancer.

The present invention also provides a method for monitoring pharmacodynamic drug action of a cdk inhibitor in a mammal comprising: performing an assay of Rb phosphorylation activity in one or more test biological samples from a mammal to which the cdk inhibitor has been administered, wherein the test biological samples were obtained from the mammal at one or more specified times after administering the cdk inhibitor; and comparing the amount of Rb phosphorylation activity in the test biological sample to that in a reference biological sample obtained from a mammal to which no cdk inhibitor has been administered, wherein the test biological sample is a peripheral blood mononuclear cell(s).
Fig. 1: Concentration Dependent Decrease in Phosphorylated Rb in HCT116 Cells 20 hours Post Treatment with COMPOUND A Determined by Western Blot Analysis
Fig. 2: Concentration Dependent Decrease in Rb in HCT116 Cells 20 hours Post Treatment with COMPOUND A Determined by ELISA
Fig. 3: Concentration Dependent Decrease in Phosphorylated Rb in MTLn3 Cells 20 hours Post Treatment with COMPOUND A Determined by Western Blot Analysis
Fig. 4: Time Course for Rb Phosphorylation in Rat Peripheral Blood Mononuclear Cells Following Ex Vivo PMA Activation
Fig. 5: Inhibition of Rb Phosphorylation in Rat PBMCs after a Single Oral Dose of COMPOUND A
Fig. 6: Inhibition of Rb Phosphorylation in MTLn3 Tumors after a Single Oral Dose of COMPOUND A
Fig. 7: COMPOUND A: Plasma and Tumor Concentrations after a Single Oral Dose
Fig. 8: Retinoblastoma Protein Phosphorylation in Human PBMCs Stimulated Ex-Vivo with PMA
Fig. 9: Inhibition of Retinoblastoma Protein Phosphorylation in Human PBMCs Treated Ex Vivo with COMPOUND A

The present invention employs, unless otherwise indicated, conventional techniques of molecular biology (including recombinant techniques), microbiology, cell biology, biochemistry and immunology, which are within the skill of the art.

As used herein, the singular form of any term also encompasses the plural form and vice-versa.

"Therapeutic response" of the present invention is defined to be any reduction in the level of the phosphorylation of the retinoblastoma protein subsequent to treatment, exposure or application of the agent that inhibits cdk activity (cdki or cdk inhibitory agent) to the tissue, cells, surrogate tissue (e.g peripheral blood mononuclear cells) of a mammal at putative phosphorylation sites, as compared to an original or pre-treatment level of the phosphorylation of the retinoblastoma protein at the putative phosphorylation sites, which indicates that inhibition of the phosphorylation of retinoblastoma protein (Rb) has occurred. The therapeutic response of the tissue and surrogate tissue of the present invention thus may be used as a correlation with the pharmacologic modulation of a tumor target.

"Exposing" a mammal or a surrogate tissue of a mammal to an agent that inhibits cdk activity, as used within the present invention, is defined to be any exposure, treatment, administration or application of at least one agent that inhibits cdk activity to the mammal or the surrogative tissue of the mammal via any process known to one of ordinary skill in the art.

"Activating" the non-proliferating cells, specifically peripheral blood mononuclear cells, of a mammal, as used within the present invention, is defined to be any exposure, treatment or application of at least one activating agent to the non-proliferating (non-cycling) cells of a mammal or surrogate tissue of a mammal, specifically the peripheral blood mononuclear cells) such that said cells become activated and thus become stimulated to enter the cell cycle. "Non-proliferating" cells are, within the scope of the present invention, defined as those cells which are non-cycling and must be activated to enter the cell cycle.

"Ex-vivo" method for testing, assessing, predicting, detecting or evaluating whether an agent that inhibits cdk activity (cdk inhibitory agent) produces a therapeutic response in a surrogate tissue of a mammal, specifically peripheral blood mononuclear cells and other non-proliferating (non-cycling) cells, is defined to be any method of the present invention which is done or carried out outside the living mammal (cells, tissue, body or organism) as known to one of ordinary skill in the art.

The agent that inhibits cdk activity (CDK inhibitor or CDK-inhibiting agent (CDKi) of the present invention may be any cdk inhibitor known to those of ordinary skill in the art (see e.g. US Patent Application 20040162303, hereby incorporated by reference) and is preferably selected from the group consisting of modified diaminopyrimidines, diaminothiazoles, diaminotriazoles and oxindoles. In one embodiment, the agent that inhibits cdk activity is a modified diaminopyrimidine and more particularly is a 4-aminopyrimidine-5-one derivative. More preferably, the agent that inhibits cdk activity is [4-amino-2-(1-methanesulfonylpiperidin-4-ylamino) pyrimidin-5-yl]-(2, 3-difluoro-6-methoxyphenyl) methanone. Other cdk inhibitors that may act as a CDKi within the scope of the present invention include purine analogues (such as CYC-202) and semi synthetic alkaloids (such as flavopiridol).

The putative phosphorylation sites of the present invention include the phosphorylation sites known to those of ordinary skill in the art and is preferably selected from the group consisting of serine 795, serine 780, serine 807/811 and threonine 821, with serine 807/811 being preferred.

The mammal of the present invention may be any mammal and is preferably selected from the group consisting of mouse, rat or human. More preferably, for the ex vivo methods described herein which are performed on a surrogate tissue which was removed from the body, the mammal is human.

The activating agent may be selected from any mitogen or activating agent known to those of ordinary skill in the art that can activate non proliferating cells, specifically peripheral blood mononuclear cells. The activating agent is preferably selected from the group consisting of PMA (phorbol 12-myristate 13-acetate), PHA (Phaseolus vulgaris) and Con A (Concanavalin A). More preferably, the activating agent is PMA.

The measuring of the level of the phosphorylation of retinoblastoma protein may be accomplished through any of the standard phosphorylation assays known to one of ordinary skill in the art, including but not limited to Western Blot, ELISA (enzyme linked immunoadsorbent assay), IHC (immunohistochemistry), and FACS (fluorescent activated cell sorting). The selection of the phosphorylation assay will depend upon the tissue, cell line or surrogate tissue sample that is utilized e.g., for example Western Blot and ELISA may be used with any or all types of tissues, cell lines or surrogate tissues whereas the IHC method would be more appropriate wherein the tissue utilized in the methods of the present invention was a tumor biopsy or buccal mucosal biopsy, whereas FACs analysis would be most applicable to samples that were single cell suspensions such as cell lines and isolated peripheral blood mononuclear cells.

The following examples are provided for illustrating purposes only and are not meant to limit the scope and/or breadth of the present invention.

### Examples

### Example 1

### Materials and Methods:

### 1.1 Cell Lines and Culture

The cell lines below (Column II, Table 1) were maintained in the designated medium (Table 1) supplemented with 10% heat-inactivated Fetal Bovine Serum (HI-FBS; GIBCO/BRL, Gaithersburg, MD) and 2 mM L-glutamine (GIBCO/BRL).

**Table 1 Cell lines used in Rb Western blot analysis: tissue origin and growth media**

| Tissue Origin | Cell Line | Growth Medium |
|---|---|---|
| human colorectal carcinoma | HCT116 | McCoys 5A |
| rat mammary adenocarcinoma | MTLn3 | RPM1 1640 |

The HCT116 cell line was obtained from the American Tissue Culture Collection (ATCC), Manassas, VA. MTLn3 cells were obtained from Hoffmann-La Roche Inc., Nutley, NJ.

### 1.2 Test Articles

The test compound, COMPOUND A, [4-amino-2-(1-methanesulfonylpiperidin-4-ylamino) pyrimidin-5-yl]-(2,3-difluoro-6-methoxyphenyl) methanone, was synthesized by Hoffmann-La Roche Inc. To prepare stock solutions, the compound was dissolved at 10 or 30 mM in 100% dimethyl sulfoxide (DMSO) (Sigma) and stored at -20°C in amber glass vials.

### 1.3 Western Blot Analysis and ELISA

Cells were washed 3 times with cold PBS on ice and scraped from the flasks. All cells were collected in 15 ml tubes. Cells were pelleted by centrifugation and lysis buffer was added (1:1 PBS: 2X Tris-Glycine SDS Sample Buffer (Invitrogen, Carlsbad, CA) containing 5% 2-β mercaptoethanol). The volume of lysis buffer used was 5 ul per 1X10⁶ cells. Samples were sonicated for 30 seconds on ice. Following sonication, samples were clarified by centrifugation at 16,000 x g for 10 minutes at 4 °C and protein concentrations were determined using Non-Interfering Protein Assay Kit (Geno Technology Incorporated, Maplewood, MO). Proteins were denatured by boiling for 5 minutes and centrifuged as described above. Proteins were resolved (20-40 ug of cell lysate per lane) by SDS-polyacrylamide gel electrophoresis using a 4-12% Tris-glycine gel (Invitrogen) and electroblotted onto a 0.2 µm PVDF membrane (Invitrogen). Membranes were blocked for about one hour at room temperature in blocking buffer (5% milk in PBS/0.1% Tween 20) followed by incubation with the primary antibody at 4°C overnight. Membranes were washed and incubated with the secondary antibody for 30 minutes at room temperature. Immunodetection was carried out using enhanced chemoluminescence (ECL Plus, Amersham Pharmacia Biotech, Piscataway, NJ). For Western blotting, total Rb was detected using antibody from Pharmingen (BD Bioscience, San Diego, CA) at a dilution of 1:2000. The phosphorylation status of Rb at serine 780, serine 795 and serine 807/811 was detected with phosphospecific antibodies at dilutions of 1:1000 (Cell Signaling Technology, Beverly, MA).

For detecting Rb phosphorylation at threonine 821 and total Rb, ELISA kits (Biosource International, Camarillo, CA) were used according to the manufacturer's protocols.

### 1.4 Analysis of Rb Phosphorylation in a Human Tumor Cell Line

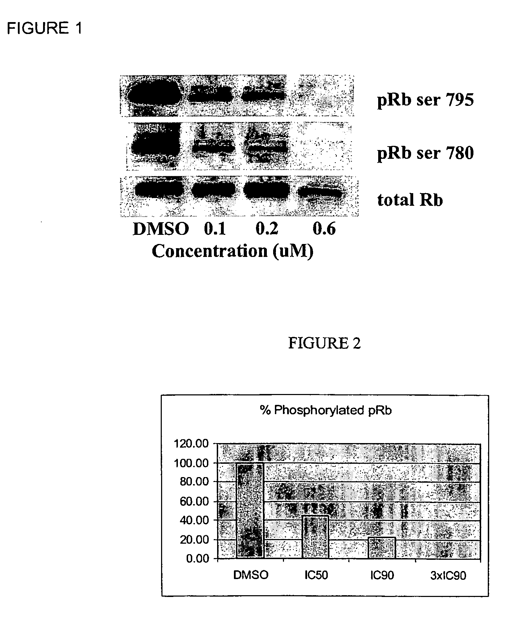

HCT116 cells were treated for 20 hours at the IC₅₀ (0.1 µM), IC₉₀ (0.2 µM) and 3XIC₉₀ (0.6 µM) concentrations for cell growth inhibition, as determined in a 5-day proliferation assay. Lysates were analyzed by Western blotting for Rb phosphorylation at serine 795 and serine 780 (Figure 1). The same lysates were tested using the human Rb ELISA kit that measures phosphorylation at threonine 821 (Figure 2). Treatment with COMPOUND A resulted in inhibition of phosphorylation at all three CDK phosphorylation sites in a concentration-dependent fashion.

### 1.5 Analysis of Rb Phosphorylation in a Rat Tumor Cell Line

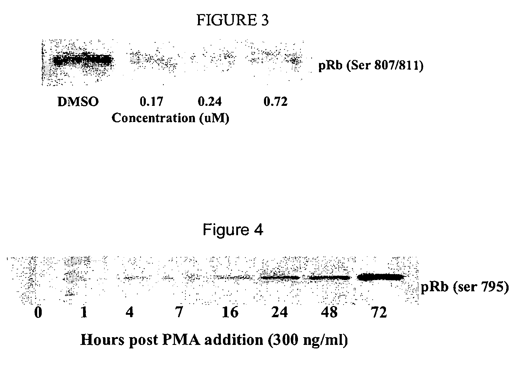

To determine if COMPOUND A could also affect the phosphorylation status of Rb from a non-human source, MTLn3 rat tumor cells were treated for 20 hours at the IC₅₀ (0.17 µM), IC₉₀ (0.24 µM) and 3XIC₉₀ (0.72 µM) concentrations for cell growth inhibition. Lysates were analyzed by Western blotting for Rb phosphorylation using polyclonal antibodies prepared against a peptide corresponding to sequences surrounding phosphorylated serine 807/811 in human Rb (Figure 3). Treatment with COMPOUND A resulted in inhibition of phosphorylation at the cross-reacting epitope in rat Rb recognized by the anti-phospho-serine 807/811 antibody.

### Example 2

### MATERIALS AND METHODS

### 2.1 Animals

Female Fischer rats obtained from Charles River Laboratories were used when they where ∼ 13-15 weeks old and weighed ∼ 150 grams. All animals were examined prior to the initiation of studies to ensure that they were healthy and acclimated to the laboratory environment. Autoclaved water and irradiated food were provided ad libitum, and the animals were maintained in a 12 hour light and dark cycle.

### 2.2 Tumors

MTLn3 cells were grown in RPMI 1640 supplemented with 10% heat-inactivated Fetal Bovine Serum (HI-FBS; GIBCO/Invitrogen, Carlsbad, CA) and 2 mM L-glutamine (GIBCO/Invitrogen) and maintained at 37°C at 5% CO₂. The MTLn3 cells were obtained from Hoffmann-La Roche Inc., Nutley, NJ. Approximately 1X10⁶ cells/0.2mls/rat in PBS (Phosphate Buffered Saline) Phenol-red free, Ca⁺² and Mg⁺² - free were implanted subcutaneously in the right mammary fat pad.

### 2.3 Test Article

The test compound COMPOUND A, [4-amino-2-(1-methanesulfonylpiperidin-4-ylamino) pyrimidin-5-yl]-(2, 3-difluoro-6-methoxyphenyl) methanone, was synthesized by Discovery Chemistry, Hoffmann-La Roche Inc. COMPOUND A was formulated as a suspension in 1% Klucel LF with 0.1% Tween 80 for oral injection. Compound was stored at 4°C and vortexed immediately prior to administration.

### 2.4 Experimental Design

The dose selected was 80 mg/kg, determined to be an efficacious dose in this animal model. Tumor growth inhibition in the MTLn3 xenograft model after 2 to 3 weeks of oral dosing at 80 mg/kg once weekly averaged 90% with 2/10 complete regressions. COMPOUND A was dosed on day 10 post implantation of cells, at which time the tumors were 100-200 mm³ in size. In this pharmacokinetic/pharmacodynamic experiment, plasma and tumors were collected at 1, 2, 6, 24 and 72 hours post dosing of a single oral dose of COMPOUND A. Tumors were removed and immediately snap frozen in liquid nitrogen and stored at -80°C until drug concentration analysis. Untreated and vehicle treated control groups were included in the analysis. Concentrations of COMPOUND A in plasma and tumors were determined in the same three animals per time point. Whole blood was also collected for analysis of Rb phosphorylation. Peripheral blood mononuclear cells (PBMCs) were isolated, stimulated ex vivo with mitogen and analyzed by Western blot as described below.

In a second pharmacodynamic study, plasma concentrations of COMPOUND A were similarly analyzed as described above, and Rb phosphorylation in both PBMCs and tumor xenografts treated with a single oral dose of COMPOUND A was examined.

### 2.5 Analysis of Rb Phosphorylation in Ex Vivo Stimulated Rat PBMCs

PBMCs are not cycling cells and need to be stimulated with the mitogen PMA to enter the cell cycle. Treatment of these cells with PMA at a concentration of 300 ng/ml for 24 hours was sufficient for detection of phosphorylated Rb in rat PBMCs by Western blot analysis (Figure 4).

### 2.6 PBMC Isolation and Stimulation

Blood from three rats at the specified times was pooled (∼10 mls total) and PBMCs were separated using the Lymphocyte Separation Medium (LSM) and protocol supplied by ICN Biomedicals Inc. (Costa Mesa, CA). Briefly whole blood was diluted 1:1 with cold HBSS (Hanks Balanced Salt Solution), layered onto the LSM and centrifuged. The layer of mononuclear cells above the LSM was collected, transferred to a new tube and washed once with cold HBSS. Cells were resuspended in RPMI 1640, 10% HI-FBS, 2 mM L-glutamine and 0.01 mg/ml Gentamicin Reagent Solution (GIBCO/Invitrogen). Cells were seeded into T-75 cm² flasks at ≤ 5 1×10⁷ cells in 20 mls medium and stimulated with 300 ng/ml of phorbol 12-myristate 13-acetate (PMA) for 24 hours. After 24 hours, cells are collected on ice, washed, resuspended in 1 ml cold PBS and processed as described below for Western blot analysis.

### 2.7 Western Blot Analysis

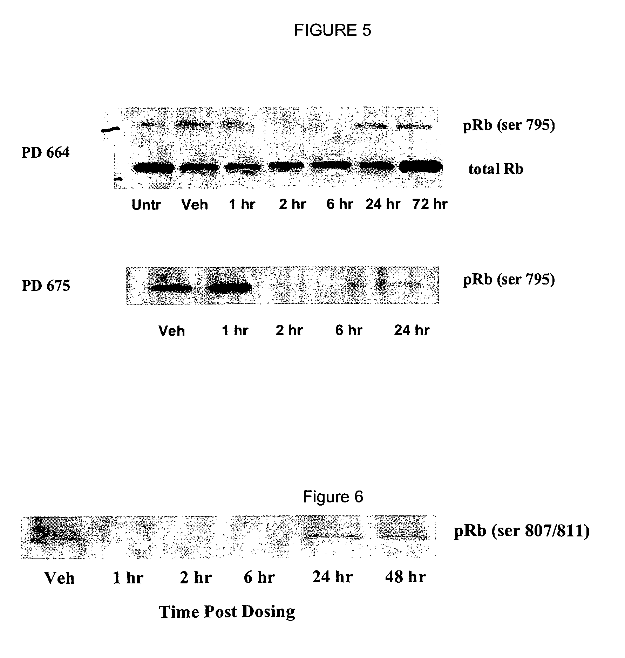

PBMCs were collected by transferring the cells, including attached cells and those in suspension, into 50 ml tubes. The flasks were placed on ice and the attached cells were scraped from the flasks and combined with the suspension cells. The cell pellets were washed twice with cold PBS. Lysis buffer was added (1:1 PBS: 2X Tris-Glycine SDS Sample Buffer (Invitrogen, Carlsbad, CA) containing 5% 2-β mercaptoethanol) to the cell pellet. The volume of lysis buffer used was 5 ul per 1×10⁶ cells. Samples were sonicated on ice for 30 seconds, clarified by centrifugation at 16,000 x g for 10 minutes at 4°C and protein concentrations determined using Non-Interfering Protein Assay Kit (Geno Technology Incorporated, Maplewood, MO). Tumor lysates were prepared by homogenizing the whole frozen tumor in lysis buffer and clarifying by centrifugation. Proteins were denatured by boiling for 5 minutes, centrifuged as described above, resolved (20-40 ug of cell lysate per lane) by SDS-polyacrylamide gel electrophoresis using a 4-12% Tris-glycine gel (Invitrogen) and electroblotted onto a 0.2 µm PVDF membrane (Invitrogen). Membranes were blocked for about 1 hr at room temperature in blocking buffer (5% milk in PBS/0.1% Tween 20) followed by incubation with the primary antibody at 4°C overnight. Membranes were washed and incubated with the secondary antibody for 30 minutes at room temperature. Immunodetection was carried out using enhanced chemoluminescence (ECL Plus, Amersham Pharmacia Biotech, Piscataway, NJ). For Western blotting, total Rb was detected using antibody from Pharmingen (BD Bioscience, San Diego, CA) at a dilution of 1:2000. PBMC lysates were analysed by Western blotting for Rb phosphorylation using a polyclonal antibody prepared against a peptide corresponding to sequence surrounding phosphorylated serine 795 in human Rb (Figure 5) and tumor lysates were analysed similarly using polyclonal antibodies prepared against a peptide corresponding to sequences surrounding phosphorylated serine 807/811 in human Rb (Figure 6). Both antibodies cross react with rat Rb. The phosphospecific antibodies were used at dilutions of 1:1000 (Cell Signaling Technology, Beverly, MA).

### 2.8 Plasma and Tumor Exposures after Oral Dosing of COMPOUND A

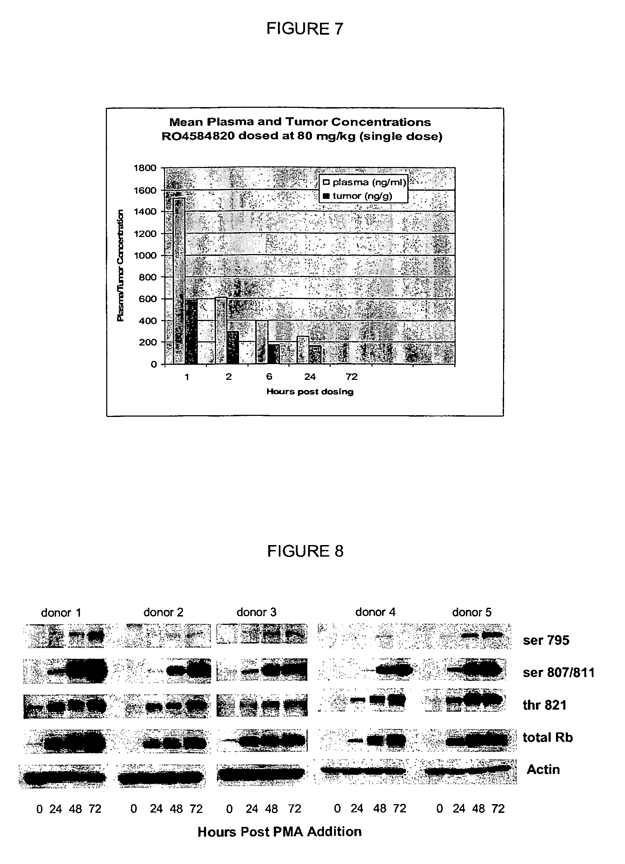

After a single oral dose of 80 mg/kg, plasma and tumor tissues show a similar concentration time profile of COMPOUND A. Maximum concentrations in both plasma and tumor tissue were observed 1 hr post dose. The concentrations in the tumor samples taken from the same animals were approximately 50% of the plasma concentration and at 24 hrs tumor drug levels (164 ± 28 ng/g) approximated plasma exposures at 24 hours (250 ± 74 ng/ml) (Figure 7). Drug levels in plasma and tumor tissue were measurable up to 24 hrs after dosing in all the animals in PD 664 (3 rats per time point). One out of three animals had a measurable plasma concentration ≥12.5 ng/ml) at 72 hr after dosing. Overall the plasma data seem fairly predictive for tumor concentrations particularly since the extraction of drug from the tumor samples might be less than complete. No drug was detectable in the vehicle group.

Similarly in PD 675, after a single 80 mg/kg oral dose of COMPOUND A, drug was measurable in all the animals up to 6 hrs (339 ± 88 ng/ml) and in 2/3 animals 24 hr and 48 hr after dosing. Mean plasma concentrations were steady from 1-24 hr. No drug was detectable in the vehicle group.

### 2.9 Analysis of Rb Phosphorylation in Rat PBMCs

Treatment of female Fischer rats with a single oral dose of 80 mg/kg COMPOUND A resulted in a decrease in Rb phosphorylation in ex vivo stimulated PBMCs (Figure 5) at 2 and 6 hours post treatment with COMPOUND A and up to 24 hours after treatment in both of the PD studies. Total Rb levels did not appear to decrease with treatment.

### 2.10 Analysis of Rb Phosphorylation in Rat MTLn3 Xenografts

In tumor xenografts COMPOUND A also shows a reduction in phosphorylation of Rb in a timecourse similar to that seen in the PBMCs (Figure 6). Each of the lanes represents a tumor isolated from one animal and for each time point three different tumors were analyzed with similar decreases in phosphorylated Rb observed. The serine 807/811 antibody was used as it gave a stronger signal in the tumor tissue than the serine 795 antibody used in the PBMC lysates.

### Example 3

### 3.1 Test Article

The test compound COMPOUND A, [4-amino-2-(1-methanesulfonylpiperidin-4-ylamino) pyrimidin-5-yl]-(2, 3-difluoro-6-methoxyphenyl) methanone, was synthesized by Discovery Chemistry, Hoffmann-La Roche Inc. To prepare stock solutions, the compound was dissolved at 10 or 30 mM in 100% dimethyl sulfoxide (DMSO) (Sigma) and stored at -20°C in amber glass vials.

### 3.2 Human PBMC Isolation and Treatment

Approximately 10 mls of blood was collected from five healthy human volunteers into sodium heparin treated Vacutainer blood collection tubes by Employee Health Services. PBMCs were separated using the Lymphocyte Separation Medium (LSM) and protocol supplied by ICN Biomedicals Inc. (Costa Mesa, CA). Briefly whole blood was diluted 1:1 with cold HBSS (Hanks Balanced Salt Solution), layered onto the LSM and centrifuged. The band of mononuclear cells above the LSM was collected, transferred to a new tube and washed once with cold HBSS. Cells were resuspended in RPMI 1640, 10% HI- FBS, 2 mM L-glutamine and 0.01 mg/ml Gentamicin Reagent Solution (GIBCO/Invitrogen). Cells were seeded into T-75 cm² flasks at ≤ 1X10⁷ cells in 20 mls medium and stimulated with 300 ng/ml of phorbol 12-myristate 13-acetate (PMA) for 24 through 72 hours. After PMA stimulation, cells are collected on ice, washed, resuspended in 1 ml cold PBS and processed as described below for Western blot analysis.

### 3.3 Western Blot Analysis

PBMCs were collected by transferring the cells, including attached cells and those in suspension, into 50 ml tubes. The flasks were placed on ice and the attached cells were scraped from the flasks and combined with the suspension cells. The cell pellets were washed twice with cold PBS. Lysis buffer was added (1:1 PBS: 2X Tris-Glycine SDS Sample Buffer (Invitrogen, Carlsbad, CA) containing 5% 2-β mercaptoethanol) to the cell pellet. The volume of lysis buffer used was 5 ul per 1X10⁶ cells. Samples were sonicated on ice for 30 seconds, clarified by centrifugation at 16,000 x g for 10 minutes at 4 °C and protein concentrations determined using Non-Interfering Protein Assay Kit (Geno Technology Incorporated, Maplewood, MO). Proteins were denatured by boiling for 5 minutes, centrifuged as described above, resolved (20-40 ug of cell lysate per lane) by SDS-polyacrylamide gel electrophoresis using a 4-12% Tris-glycine gel (Invitrogen) and electroblotted onto a 0.2 µm PVDF membrane (Invitrogen). Membranes were blocked for about 1 hr at room temperature in blocking buffer (5% milk in PBS/0.1% Tween 20) followed by incubation with the primary antibody at 4°C overnight. Membranes were washed and incubated with the secondary antibody for 30 minutes at room temperature. Immunodetection was carried out using enhanced chemoluminescence (ECL Plus, Amersham Pharmacia Biotech, Piscataway, NJ). For Western blotting, total Rb was detected using antibody from Pharmingen (BD Bioscience, San Diego, CA) at a dilution of 1:2000. Lysates were analysed by Western blotting for Rb phosphorylation using polyclonal antibodies against phosphorylated serine 795, serine 780, threonine 821 and serine 807/811 in human Rb (Figure 8). The phosphospecific antibodies were used at dilutions of 1:1000 (Cell Signaling Technology, Beverly, MA).

### 3.4 Analysis of Retinoblastoma Phosphorylation in Ex Vivo Stimulated Human PBMCs

PBMCs are not cycling cells and therefore need to be stimulated to enter the cell cycle. PMA, at a concentration of 300 ng/ml for 24 hours, is sufficient for detection of phosphorylated Rb in rat PBMCs by Western blot analysis. Human PBMCs were isolated, stimulated ex vivo with 300 ng/ml PMA and threonine 821, serine 780, serine 795, and serine 807/811 phosphorylation sites on Rb were evaluated by Western blotting at 24, 48 and 72 hours post stimulation. Significant phosphorylation at serine 807/811 and threonine 821 sites was detected at 24 hours post mitogen addition in human PBMCs from all five human donors (Figure 8). While phosphorylation at serine 795 (Figure 8) and serine 780 (data not shown) was also detected, it was not significant until 48-72 hours post PMA stimulation. Total Rb and actin were run for standardization of protein loading.

### 3.5 Analysis of Retinoblastoma Phosphorylation in Human PBMCs Treated Ex-Vivo with COMPOUND A

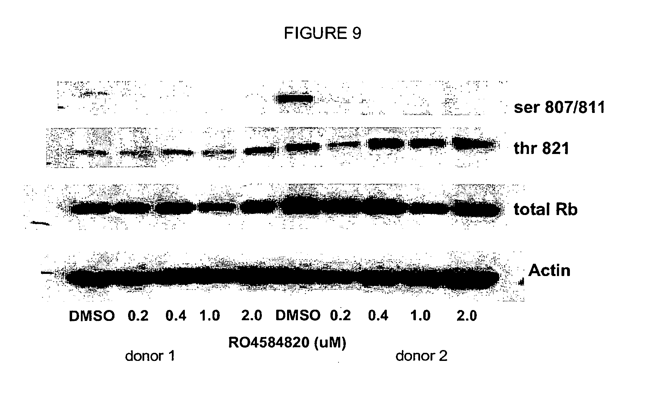

PBMCs from two healthy volunteers were isolated and stimulated ex vivo with PMA as described above and simultaneously incubated with COMPOUND A at 0.2 µm through 2.0 µM, concentrations that represent the IC₉₀ through 10 X IC₉₀ concentrations for tumor cell growth inhibition, as determined in a 5-day proliferation assay. Lysates were analyzed by Western blotting at the serine 807/811 and threonine 821 sites on Rb (Figure 9). Treatment with COMPOUND A resulted in a complete inhibition of phosphorylation at the serine 807/811 site in both donors at all concentrations. Phosphorylation at the threonine 821 site, after 24 hours incubation with COMPOUND A, was not inhibited at any concentration.

The foregoing description illustrates and describes the methods of the present disclosure. Additionally, the disclosure shows and describes only certain embodiments of the methods but, as mentioned above, it is to be understood that the teachings of the present disclosure are capable of use in various other combinations, modifications, and environments and is capable of changes or modifications within the scope of the inventive concept as expressed herein, commensurate with the above teachings and/or the sill or knowledge of the relevant art. The embodiments described hereinabove are further intended to explain best modes known of practicing the invention and to enable others skilled in the art to utilize the invention in such, or other, embodiments and with the various modifications required by the particular applications or uses of the invention. Accordingly, the description is not intended to limit the invention to the form(s) disclosed herein.

## Claims

1. An ex-vivo method for testing whether a mammal will respond therapeutically to a method of treating cancer comprising administering an agent that inhibits cdk activity, wherein the testing method comprises: a) activating a first amount of peripheral blood mononuclear cells of a mammal; b) measuring the level of the phosphorylation of retinoblastoma protein (Rb) at putative phosphorylation sites in the first amount of peripheral blood mononuclear cells of the mammal; c) activating a second amount of peripheral blood mononuclear cells of a mammal and simultaneously exposing the second amount of peripheral blood mononuclear cells of the mammal to the agent that inhibits cdk activity; d) measuring the level of the phosphorylation of retinoblastoma protein (Rb) at the putative phosphorylation sites in the second amount of peripheral blood mononuclear cells of the mammal, wherein a difference in the level of the phosphorylation of retinoblastoma protein measured in step d) compared to the level of the phosphorylation of retinoblastoma protein measured in step b) indicates that the mammal will respond therapeutically to the method of treating cancer.

2. The method of claim 1, wherein the agent that inhibits cdk activity is a diaminopyrimidine.

3. The method of claim 2, wherein the agent that inhibits cdk activity is [4-amino-2-(1-methanesulfonylpiperidin-4-ylamino) pyrimidin-5-yl]-(2, 3-difluoro-6-methoxyphenyl) methanone.

4. The method of claims 1- 3, wherein the putative phosphorylation sites are selected from the group consisting of serine 795, serine 780, serine 807/811 and threonine 821.

5. The method of claim 4, wherein the putative phosphorylation site is serine 807/811.

6. A method for monitoring pharmacodynamic drug action of a cdk inhibitor in a mammal comprising: performing an assay of Rb phosphorylation activity in one or more test biological samples from a mammal to which the cdk inhibitor has been administered, wherein the test biological samples were obtained from the mammal at one or more specified times after administering the cdk inhibitor; and comparing the amount of Rb phosphorylation activity in the test biological sample to that in a reference biological sample obtained from a mammal to which no cdk inhibitor has been administered, wherein the test biological sample is a peripheral blood mononuclear cell(s).

7. The method of claim 6, wherein the mammal is a human.

## Patentansprüche

1. Ex∼vivo∼Verfahren, um zu testen, ob ein Säuger therapeutisch auf ein Verfahren zur Be-handlung von Krebs anspricht, das das Verabreichen eines Mittels, das die cdk~Aktivität inhibiert, umfaßt, wobei das Testverfahren a) das Aktivieren einer ersten Menge peripherer mononukleärer Blutzellen eines Säugers; b) das Messen des Phosphorylierungsgrades von Retinoblastom-Protein (Rb) an putativen Phosphorylierungsstellen in der ersten Menge peripherer mononukleärer Blutzellen des Säugers; c) das Aktivieren einer zweiten Menge peripherer mononukleärer Blutzellen eines Säugers und gleichzeitig das Aussetzen der zweiten Menge peripherer mononuklearer Blutzellen des Säugers einem Mittel, das die cdk∼Aktivität inhibiert; d) das Messen des Phosphorylierungsgrades von Retinoblastom-Protein (Rb) an den putativen Phosphorylierungsstellen in der zweiten Menge peripherer mononukleärer Blutzellen des Säugers umfaßt, wobei eine Differenz in bezug auf den Phosphorylierungsgrad von Retinoblastom-Protein, gemessen in Schritt d), im Vergleich zu dem Phosphorylierungsgrad von Retinoblastom-Protein, gemessen in Schritt b), angibt, daβ der Säuger therapeutisch auf das Verfahren zur Behandlung von Krebs ansprechen wird.

2. Verfahren nach Anprusch 1, wobei das Mittel, das die cdk-Aktivität inhibiert, Diaminopyrimidin ist.

3. Verfahren nach Anprusch 2, wobei das Mittel, das die cdk-Aktivität inhibiert, [4- Amino-2-(1-methansulfonylpiperidin-4-ylamino)pyrimidin-5-yl]-(2,3-difluor-6-methoxyphenyl)-methanon ist.

4. Verfahren nach den Ansprüchen 1-3, wobei die putativen Phosphorylierungsstellen aus der Gruppe ausgewählt sind, bestehend aus Serin 795, Serin 780, Serin 807/811 und Treonin 821.

5. Verfahren nach Anspruch 4, wobei die putative Phosphorylierungsstellen Serin 807/811 ist.

6. Verfahren zum Überwachen der pharmakodynamischen Arzneimittelwirkung eines cdk-Inhibitors bei einem Säuger, umfassend; die Durchführung eines Assays der Rb-Phosphorylierungsaktivität in einer oder mehreren biologischen Untersuchungsproben von einem Säuger, dem der cdk-Inhibitor verabreicht wurde, wobei die biologischen Untersuchungsproben von einem Säuger zu einem oder mehren speziellen Zeitpunkt(en) nach der Verabreichung des cdk-Inhibitors erhalten wurden; und das Vergleichen des Ausmaßes der Rb-Phosphorylierungsaktivität in der biologischen Untersuchungsprobe mit dem in einer biologischen Vergleichsprobe, die von einem Säuger erhalten wurde, dem kein cdk-Inhibitor verabreicht wurde, wobei die biologische Untersuchungsprobe (eine) periphere mononukleäre Blutzelle(n) ist.

7. Verfahren nach Anspruch 6, wobei der Säuger ein Mensch ist.

## Revendications

1. Procédé ex-vivo pour tester si un mammifère va repondre sur le plan thérapeutique à un procédé pour le traitement du cancer, comprenant l'administration d'un agent qui inhibe l'activité cdk, dans lequel le procédé de test comprend les opérations consistant à:
a) activer une première quantité de cellules mononucléaires du sang périphérique d'un mammifère ;
b) mesurer le niveau de phosphorylation de la protéine rétinoblastome (Rb) à des sites de phosphorylation putatifs dans la première quantité de cellules mononucléaires du sang phériphérique du mammifère ;
c) activer une seconde quantité de cellules mononucléaires du sang périphérique d'un mammifère et, simultanément, exposition de la seconde quantité de cellules mononucléaires du sang périphérique du mammifère à l'agent qui inhibe qui inhibe l'activité cdk ;
d) mesurer le niveau de phosphorylation de la protéine rétinoblastome (Rb) aux sites de phosphorylation putatifs dans la seconde quantité de cellules mononucléaires de sang périphérique du mammifère, dans lequel une différence du niveau de phosphorylation de la protéine de rétinoblastome mesurée à l'étape d), comparée au niveau de la phosphorylation de la protéine de rétinoblastome mesurée à l'étape b), indique que le mammifère va répondre sur le plan thérapeutique au procédé de traitement du cancer.

2. Procédé selon la revendication 1, dans lequel l'agent qui inhibe l'activité cdk est une pyrimidine diaminée.

3. Procédé selon la revendication 2, dans lequel l'agent qui inhibe l'activité cdk est la [4-amino-2-(1-méthanesulfonylpipéridine-4-ylamino)pyrimidine-5-yl]-(2,3-difluoro-6-méthoxyphényl)méthanone.

4. Procédé selon la revendication 1-3, dans lequel les sites de phosphorylation putatifs sont choisis dans le groupe consistant en sérine 795, en sérine 780, en sérine 807/811 et en thréonine 821.

5. Procédé selon la revendication 4, dans lequel le site de phosphorylation putatif est la sérine 807/811.

6. Procédé pour surveiller l'action médicamenteuse pharmacodynamique d'un inhibiteur cdk chez un mammifère, comprenant les étapes consistant à :
effectuer un test de l'activité de phosphorylation Rb dans un ou plusieurs échantillon biologiques de test à partir d'un mammifère auquel on a administré l'inhibiteur cdk, dans lequel les échantillons biologiques de test ont été obtenus à partir du mammifère une ou plusieurs fois spécifiées après administration de l'inhibiteur cdk ; et
à comparer la quantité de l'activité de phosphorylation Rb dans l'échantillon biologique test avec celui d'un échantillon biologique de référence obtenu à partir d'un mammifère auquel aucun inhibiteur cdk n'a été administré, dans lequel l'échantillon biologique test est une cellule ou des cellules mononucléaires du sang périphérique.

7. Procédé selon la revendication 6, dans lequel le mammifère est un humain.


### Sequence listing

<110> F. Hoffmann-La Roche AG
<120> Biomarker for Response Monitoring of CDK Inhibitors in the Clinic
<130> case 22867
<160> 1
<170> PatentIn version 3.3
<210> 1
   <211> 928
   <212> PRT
   <213> Homo sapiens
<220>
   <221> Rb protein
   <222> (1)..(928)
   <223> GenBank L11910
<400> 1